(19) **Europäisches Patentamt**
**European Patent Office**
**Office européen des brevets**

(11) **EP 3 572 062 B1**

(12) **EUROPEAN PATENT SPECIFICATION**

(45) Date of publication and mention
of the grant of the patent:
**29.03.2023 Bulletin 2023/13**

(21) Application number: **18742325.6**

(22) Date of filing: **17.01.2018**

(51) International Patent Classification (IPC):
***A61K 9/48*** *(2006.01)*      ***A61J 3/07*** *(2006.01)*

(52) Cooperative Patent Classification (CPC):
**A61K 9/4866; A61J 3/074; A61J 3/077;**
**A61K 9/485; A61K 9/4858; A61K 9/4891**

(86) International application number:
**PCT/JP2018/001285**

(87) International publication number:
**WO 2018/135551 (26.07.2018 Gazette 2018/30)**

(54) **WATER-CONTAINING CAPSULE AND METHOD FOR PRODUCING WATER-CONTAINING CAPSULE**

WASSERHALTIGE KAPSEL UND VERFAHREN ZUR HERSTELLUNG EINER WASSERHALTIGEN KAPSEL

CAPSULE CONTENANT DE L'EAU ET MÉTHODE DE PRODUCTION DE CAPSULE CONTENANT DE L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2017   JP 2017006247**
**28.04.2017   JP 2017090818**

(43) Date of publication of application:
**27.11.2019   Bulletin 2019/48**

(73) Proprietor: **Morishita Jintan Co., Ltd.**
**Osaka-shi**
**Osaka 540-8566 (JP)**

(72) Inventors:
• **NAGAE, Kentaro**
**Hirakata-shi**
**Osaka 573-0128 (JP)**
• **NISHIKAWA, Takehiro**
**Hirakata-shi**
**Osaka 573-0128 (JP)**

• **ISHII, Katsutoshi**
**Hirakata-shi**
**Osaka 573-0128 (JP)**
• **TAGAWA, Daisuke**
**Hirakata-shi**
**Osaka 573-0128 (JP)**
• **HASHIMOTO, Taku**
**Hirakata-shi**
**Osaka 573-0128 (JP)**

(74) Representative: **Lavoix**
**Bayerstraße 83**
**80335 München (DE)**

(56) References cited:
**EP-A1- 2 749 272      EP-A1- 2 832 352**
**JP-A- S61 225 115     JP-A- 2000 336 028**
**JP-B1- 5 989 953**

**Description**

FIELD

The present disclosure relates to water-containing capsules and to a method of producing water-containing capsules.

BACKGROUND

Capsules containing contents such as functional foods and drugs have undergone various design improvements from the viewpoint of their holding functions.

PTL 1, for example, discloses a capsule formulation that can hold hydrophilic substances stably for long periods, and allows components that are contraindicated for mixing, to be contained in the same capsule.

In the capsule formulation described in PTL 1, capsule contents which include an aqueous solution enclosed in an oil-based substance containing a porous fine particle powder, are encapsulated by a hydrophilic film.

PTL 2 discloses a three-layer capsule having an aqueous core, a lipophilic intermediate layer and a hydrophilic outer layer which may contain solid particles; as well as a multiple-nozzle process ("dropping method") for producing a seamless capsule.

[CITATION LIST]

[PATENT LITERATURE]

    [PTL 1] International Patent Publication No. WO2015/115072

    [PTL 2] European Patent Application No. EP 2 749 272 A1

SUMMARY

[TECHNICAL PROBLEM]

The capsule formulation described in PTL 1 prevents the hydrophilic film, which forms part of the capsule walls, from softening by the hydrophilic substance serving as the core substance, and the feature of being able to "hold hydrophilic substances stably for long periods" mentioned in PTL 1 means that the capsule walls are resistant to softening. However, even though the capsule walls are resistant to softening in the capsule formulation described in PTL 1, the capsule walls themselves have not been excellent in terms of minimizing transpiration of water molecules.

It is therefore an object of the present disclosure to provide water-containing capsules with excellent water-transpiration resistance.

[SOLUTION TO PROBLEM]

The present inventors have devised a water-containing capsule comprising a water-containing layer that includes water, an inner layer situated on an outer side of the water-containing layer and an outer layer situated on an outer side of the inner layer, wherein the inner layer is configured by including a lipophilic material, and the outer layer is configured by including a hydrophilic material in which lipophilic particles or water-insoluble particles are dispersed.

[ADVANTAGEOUS EFFECTS OF INVENTION]

The water-containing capsule of the present disclosure has excellent water-transpiration resistance.

BRIEF DESCRIPTION OF DRAWINGS

    FIG. 1 is a diagram illustrating a water-containing capsule 1 according to a first embodiment.
    FIG. 2 is a schematic view of a production apparatus 101 for water-containing capsules.

FIG. 3 is a magnified cross-sectional view of the vicinity of the injection nozzle 103.

DESCRIPTION OF EMBODIMENTS

Specifically, the present disclosure relates to the following aspects.

[Aspect 1]

A water-containing capsule comprising a water-containing layer that includes water, an inner layer situated on an outer side of the water-containing layer and an outer layer situated on an outer side of the inner layer, wherein

the inner layer is configured by including a lipophilic material, and
the outer layer is configured by including a hydrophilic material in which lipophilic particles or water-insoluble particles are dispersed, and
the lipophilic material includes at least one of fats and oils, fatty acid, sugar fatty acid ester, aromatic hydrocarbon, chain ether, higher fatty acid ester, higher alcohol, and hydrogenated form of the foregoing, and components for the water -insoluble particles include at least one of metal oxide, metal carboxylate, and clay mineral.

In the water-containing capsule, the inner layer is configured by including a lipophilic material having an excellent water barrier property, while the outer layer is configured by including a hydrophilic material in which lipophilic particles or water-insoluble particles are dispersed. In order for water molecules having reached the outer layer from the inner layer to pass through the outer layer and transpire outward, it is necessary for them to avoid the lipophilic particles or water-insoluble particles in the outer layer that inhibit permeation of the water molecules, and this creates a more complex route for passage through the outer layer. In other words, the lipophilic particles or water-insoluble particles are components that give the outer layer a property of increased complexity for the route of water molecules through the outer layer (hereunder also referred to as "route complexity"). Since the outer layer includes lipophilic particles or water-insoluble particles as components that provide the outer layer with route complexity, water molecules have greater difficulty passing through the outer layer. As a result, the inner layer and outer layer of the water-containing capsule help prevent transpiration of water in the water-containing layer. Throughout the present specification, "preventing transpiration of water" may also be referred to as "excellent water-transpiration resistance".
Since the inner layer and outer layer of the water-containing capsule of the disclosure have excellent water-transpiration resistance, the proportion of the inner layer and outer layer in the water-containing capsule can be relatively reduced, allowing the proportion of water-containing layer to be relatively increased.
In one embodiment, the inner layer further includes solid particles, and the solid particles are dispersed in the lipophilic material.
In the water-containing capsule, the inner layer is configured by including a lipophilic material in which solid particles are dispersed, or in other words, solid particles with an excellent property of attracting water molecules and inhibiting diffusion of water molecules (this property will hereunder be referred to as "water molecule-diffusion inhibition"), are dispersed in a lipophilic material having an excellent water barrier property, and therefore water in the water-containing layer is less able to pass through the inner layer. As a result, the water-containing capsule has excellent transpiration resistance for water in the water-containing layer.
In a further embodiment, the solid particles are nonporous particles.
Since the solid particles in the water-containing capsule are nonporous particles, the water molecule-diffusion inhibition is more readily exhibited than when the solid particles are not nonporous particles, while the solid particles are also less likely to be hydrophilicized and consequently the water barrier property of the inner layer is less likely to be reduced. As a result, the water-containing capsule has excellent transpiration resistance for water in the water-containing layer.
In a further embodiment, the water-containing layer further includes a component that lowers a water vapor pressure.
Since the water-containing layer in the water-containing capsule further includes a component that lowers the water vapor pressure, the water vapor pressure in the water-containing layer is more easily lowered, and water in the water-containing layer is less able to pass through the inner layer and outer layer. As a result, the water-containing capsule has excellent transpiration resistance for water in the water-containing layer.
In a further embodiment, the outer layer includes the lipophilic particles or the water-insoluble particles in a proportion of 1 to 30 mass%.
Since the outer layer in the water-containing capsule includes lipophilic particles or water-insoluble particles in a prescribed proportion, it is easier to ensure the route complexity of the outer layer while retaining the physical properties of the outer layer.

In a further embodiment, the lipophilic particles or the water-insoluble particles have a mean particle diameter of 1 to 120 μm.

Since the lipophilic particles or water-insoluble particles in the water-containing capsule have a prescribed mean particle diameter, the lipophilic particles or water-insoluble particles diffuse more easily in the hydrophilic material, while the route complexity of the outer layer can also be more easily ensured and the physical properties of the outer layer can be more easily retained.

In a further embodiment, the outer layer includes the lipophilic particles or the water-insoluble particles in a number density of 300 to 700/mm$^2$.

Since the outer layer in the water-containing capsule includes the lipophilic particles or water-insoluble particles in a prescribed number density, it is easier to ensure the route complexity of the outer layer and also easier to retain the physical properties of the outer layer.

According to the invention, there is also provided a method of producing the water-containing capsule according to the invention , the method comprising:

a step of preparing a first nozzle, a second nozzle disposed so as to surround the first nozzle on an outer side of the first nozzle, and a third nozzle disposed so as to surround the second nozzle on an outer side of the second nozzle,

a step of ejecting a material that is to form the water-containing layer, a heated material that is to form the inner layer and a heated material that is to form the outer layer, from the first nozzle, the second nozzle and the third nozzle, respectively, to form a water-containing pre-capsule, and

a step of releasing the water-containing pre-capsule into a cooling solvent so as to cool the water-containing pre-capsule.

This production method allows easy production of the water-containing capsules described above.

The water-containing capsules and method of producing water-containing capsules of the present disclosure will now be described in detail.

[Water-containing capsules]

The water-containing capsules of the disclosure include a water-containing layer that includes water, an inner layer situated on the outer side of the water-containing layer and an outer layer situated on the outer side of the inner layer.

<Inner layer>

The inner layer includes a lipophilic material. The lipophilic material inhibits transpiration of water in the water-containing layer to the outside of the water-containing capsules, due to the low permeability for water molecules provided by its high gas barrier property. Throughout the present specification, the concept of low permeability for water molecules may be referred to as "water barrier property".

The lipophilic materials include fats and oils (for example, glycerin fatty acid esters such as safflower oil, linseed oil, sesame oil, olive oil, soybean oil, mustard oil, rapeseed oil, corn oil, castor oil, evening primrose oil, palm kernel oil, jojoba oil, cottonseed oil, coconut oil, peanut oil and cacao oil), fatty acids, sugar fatty acid esters (for example, sucrose fatty acid esters), hydrocarbons (for example, terpenes, paraffins and beeswax), aromatic hydrocarbons, chain ethers, higher fatty acid esters, higher alcohols, and hydrogenated forms of the foregoing (for example, hydrogenated fats or oils such as margarine and shortening).

The lipophilic material may be either solid or viscous, so long as it can function as the inner layer for the water-containing capsules, or it may be liquid so long as it becomes solid or viscous by dispersion of the solid particles.

The melting point of the lipophilic material is not particularly restricted and may be a melting point of 20°C or higher, preferably 30°C or higher and more preferably 40°C or higher, and preferably a melting point of no higher than 100°C, from the viewpoint of retaining the shapes of the water-containing capsules at the temperature of usage.

Optionally, the inner layer further includes solid particles, the solid particles preferably being dispersed in the lipophilic material. The solid particles have excellent water molecule-diffusion inhibition, and act to cause water molecules having reached the inner layer to be retained in the inner layer without migrating to the outer layer.

The solid particles are preferably nonporous particles. The nonporous particles have an specific surface area of preferably less than 100 m$^2$/g, more preferably no greater than 50 m$^2$/g, even more preferably no greater than 40 m$^2$/g, yet more preferably no greater than 30 m$^2$/g and most preferably no greater than 20 m$^2$/g. If the solid particles have a specific surface area in this range the solid particles will be less likely to excessively adsorb water, thus making the solid particles less likely to be hydrophilicized so that the inner layer will be unlikely to have a reduced water barrier property. An increased specific surface area will cause a greater absolute amount of water molecules to be adsorbed by each of the solid particles, often hydrophilicizing the solid particles and resulting in a lower water barrier property of the inner layer.

The specific surface area is measured according to JIS Z 8830:2013, "Determination of the Specific Surface Area of Powders (Solids) by Gas Adsorption". The method of measuring the amount of adsorption gas is not particularly restricted and may be a static capacity method, a continuous capacity method, a weighing method or a carrier gas method, with a static capacity method being preferred.

Examples of components for the solid particles include publicly known metal oxides (for example, silicas such as amorphous synthetic silica, alumina, alumina hydrate and titanium dioxide), metal carbonates (for example, calcium carbonate and magnesium carbonate), metal carboxylates (for example, alkali metal carboxylates, and alkaline earth metal carboxylates such as magnesium stearate), clay minerals and polymers (such as polypropylene).

The lipophilic material in the inner layer may also include components other than the solid particles, in ranges such that the effect of the invention is still exhibited. Any components other than solid particles may be dissolved or dispersed in the lipophilic material.

The solid particles have a mean particle diameter of preferably 1 to 120 $\mu$m, more preferably 5 to 100 $\mu$m and even more preferably 10 to 80 $\mu$m. If the solid particles have a mean particle diameter within this range, the total number of solid particles, and therefore the total surface area of the solid particles, will be increased compared to particles having a larger mean particle diameter than this mean particle diameter range, given the same proportion of solid particles in the inner layer, and consequently the solid particles will more readily exhibit water molecule-diffusion inhibition. Moreover, if the solid particles have a mean particle diameter within this range, the individual sizes of the solid particles will be smaller than particles having a larger mean particle diameter than this mean particle diameter range, given the same proportion of solid particles in the inner layer, and consequently the water barrier property of the inner layer will be less likely to be lowered even when the solid particles have attracted water molecules. As a result, the solid particles will more easily exhibit water molecule-diffusion inhibition while the water barrier property of the lipophilic material will be retained.

When the inner layer includes solid particles, the inner layer preferably includes the solid particles at 1 to 30 mass%, more preferably 3 to 25 mass% and even more preferably 5 to 20 mass%, based on the total mass of the inner layer. If the inner layer includes the solid particles in this proportion, the solid particles will exhibit water molecule-diffusion inhibition, while the water barrier property of the inner layer will be unlikely to be lowered.

Throughout the present specification, the particle diameters of the water-containing capsules, solid particles and lipophilic particles or water-insoluble particles (hereunder also referred to collectively as "particles") are the circle equivalent diameters as calculated from the projected area in a projection image, and the mean particle diameters of the particles are the median diameter ($^{50}D_p$) of the particle size distribution (based on quantity).

Specifically, the mean particle diameter of the particles is measured in the following manner.

(1) A digital microscope by Keyence Corp. (trade name: VHX-2000, zoom lens: $\times$20 to $\times$200) and its included software are prepared, with a constant temperature and humidity room at temperature: 20$\pm$5°C and humidity: 65$\pm$5% RH.
(2) A particle sample is allowed to stand in the constant temperature and humidity room for 3 hours.
(3) The sample is photographed with a digital microscope.
(4) The particle projected area (A) is measured with the included software and the circle equivalent diameter of each particle (CD) is calculated by the following formula:

[Formula 1]

$$CD = 2\sqrt{\frac{A}{\pi}}$$

(5) The included software is used to create a particle size distribution (based on quantity) for the circle equivalent diameter CD of 1,000 particles, and the median diameter ($^{50}D_p$) is determined from the particle size distribution (based on quantity) and recorded as the mean particle diameter of the particles.

<Outer layer>

The outer layer is configured by including a hydrophilic material in which lipophilic particles or water-insoluble particles are dispersed. The hydrophilic material is a component that increases the strength of the water-containing capsules and thus helps to maintain their shapes, while it also has the function of improving the storage life of the water-containing capsules. The lipophilic particles or water-insoluble particles are components that provide the outer layer with route complexity.

Examples of hydrophilic materials include gelatin, carrageenan, agar, sodium alginate, pullulan, glucomannan, gum arabic, furcellaran, *Eucheuma* algae, gellan gum, hydroxypropyl methyl cellulose, hydroxypropyl cellulose, polyvinylpyrrolidone, polyvinyl alcohol, carboxymethyl cellulose, locust bean gum, pectin, xanthan gum and starch.

Examples of components for the lipophilic particles that may be dispersed in the hydrophilic material include plant waxes, animal waxes, mineral waxes, petroleum waxes, fats and oils, fatty acids, lecithin and hydrocarbons (for example, terpenes, paraffins and beeswax).

Examples of fats and oils include vegetable oils and animal oils. Examples of vegetable oils include rapeseed oil, corn oil, soybean oil, cottonseed oil, safflower oil, palm oil, coconut oil, rice bran oil, sesame oil, cocoa butter, olive oil and palm kernel oil. Examples of animal oils include fish oil, lard, beef tallow, chicken fat and milk fat.

The water-insoluble particle components that may be dispersed in the hydrophilic material include metal oxides (for example, silicon dioxide such as particulate silicon dioxide), metal carboxylates (for example, alkali metal carboxylates and alkaline earth metal carboxylates such as magnesium stearate), clay minerals (for example, nano clay, and specifically kaolinite, smectite, sericite, illite, glauconite, montmorillonite, chlorite and zeolite nano clay).

The lipophilic particles or water-insoluble particles have a mean particle diameter of preferably 1 to 120 $\mu$m, more preferably 5 to 100 $\mu$m and even more preferably 10 to 80 $\mu$m. If the lipophilic particles or water-insoluble particles have a mean particle diameter within this range, the lipophilic particles or water-insoluble particles will more readily disperse in the hydrophilic material and the route complexity of the outer layer can be more easily ensured, while the physical properties of the outer layer will also be more easily maintained.

According to the invention, the outer layer includes such lipophilic particles or water-insoluble particles, and the outer layer preferably includes them at 1 to 30 mass%, more preferably 3 to 25 mass% and even more preferably 5 to 20 mass%, based on the total mass of the inner layer. If the outer layer includes the lipophilic particles or water-insoluble particles in this proportion, it will be easier to ensure the route complexity of the outer layer and also easier to retain the physical properties of the outer layer.

The outer layer preferably includes the lipophilic particles or water-insoluble particles at a number density of 100 to 2,000/mm$^2$, more preferably 200 to 1,000/mm$^2$ and even more preferably 300 to 700/mm$^2$. If the outer layer includes the lipophilic particles or water-insoluble particles in this number density, it will be easier to ensure the route complexity of the outer layer and also easier to retain the physical properties of the outer layer.

The number density is measured in the following manner.

(1) The perimeter of a water-containing capsule is cut on a prescribed plane so as to include only the outer layer, to prepare a sample.
(2) The sample is placed on slide glass and another cover glass is placed over it to create a preparation.
(3) The sample is observed with a microscope at a focal depth of 5 $\mu$m, and the number of lipophilic particles or water-insoluble particles within a prescribed area is counted.
(4) The number of lipophilic particles or water-insoluble particles is divided by the prescribed area to calculate the number density for the sample.
(5) The measurement is repeated a total of 10 times using different water-containing capsules, and the average value is used as the number density for the water-containing capsules.

An example for the microscope is a digital microscope by Keyence Corp. (trade name: VHX-2000, zoom lens: $\times$20 to $\times$200).

The hydrophilic material in the outer layer may also include components other than the lipophilic particles or water-insoluble particles, in ranges such that the effect of the invention is still exhibited. Any components other than lipophilic particles or water-insoluble particles may be dissolved or dispersed in the hydrophilic material.

<Water-containing layer>

The water-containing layer is not particularly restricted so long as it contains water, and it may even be composed entirely of water.

The water-containing layer may contain components that lower the water vapor pressure, from the viewpoint of inhibiting transpiration of the water in the water-containing layer. If the water-containing layer includes components that lower the water vapor pressure, the vapor pressure of the water in the water-containing layer will decrease, thereby inhibiting transpiration of the water in the water-containing layer.

Examples of components that can lower the water vapor pressure include electrolytes and thickeners.

Electrolytes include halides, sulfates, nitrates and phosphates of alkali metals and alkaline earth metals, examples of which are sodium chloride, calcium chloride, potassium chloride and sodium phosphate.

Examples of thickeners include gelatin, carrageenan, agar, sodium alginate, pullulan, glucomannan, gum arabic, furcellaran, *Eucheuma* algae, gellan gum, hydroxypropyl methyl cellulose, hydroxypropyl cellulose, polyvinylpyrrolidone, polyvinyl alcohol, carboxymethyl cellulose, locust bean gum, pectin, xanthan gum and starch.

When the water-containing layer includes a component that lowers the water vapor pressure, the amount of the component that lowers the water vapor pressure is not particularly restricted so long as it is in a range allowing the vapor pressure of water in the water-containing layer to be lowered, but the water-containing layer preferably contains the water vapor pressure-lowering component at 1 to 30 mass% and more preferably 5 to 20 mass%, based on the total mass of the water-containing layer.

The water-containing layer may also contain components other than the water vapor pressure-lower component, in a range such that the effect of the disclosure is still exhibited.

<Water-containing capsules>

The water-containing capsules include the inner layer and the outer layer at proportions of preferably 15 to 50 mass% and 5 to 50 mass%, more preferably 20 to 45 mass% and 5 to 30 mass% and even more preferably 25 to 40 mass% and 5 to 20 mass%, respectively, based on the total mass of the water-containing capsules. This will allow the water in the water-containing layer to be held in a state such that it is less able to pass through the inner layer and outer layer.

When the water-containing capsules are composed of a water-containing layer, an inner layer and an outer layer, the water-containing capsules include the water-containing layer, the inner layer and the outer layer at proportions of preferably 10 to 80 mass%, 15 to 50 mass% and 5 to 50 mass%, more preferably 20 to 75 mass%, 20 to 45 mass% and 5 to 35 mass% and even more preferably 40 to 70 mass%, 25 to 40 mass% and 5 to 20 mass%, respectively, based on the total mass of the water-containing capsules. This will allow the water in the water-containing layer to be held in a state such that it is less able to pass through the inner layer and outer layer.

The water-containing capsules may have any desired mean particle diameter, depending on the purpose of use, such as a mean particle diameter of 3.0 to 6.0 mm, for example.

The water-containing capsules may also include any layers other than the water-containing layer, inner layer and outer layer, so long as the effect of the invention is still exhibited.

The water-containing capsules of the disclosure may also be seamless capsules. Specifically, the inner layer and outer layer may both be seamless capsules. Seamless capsules can be produced by a known dropping method.

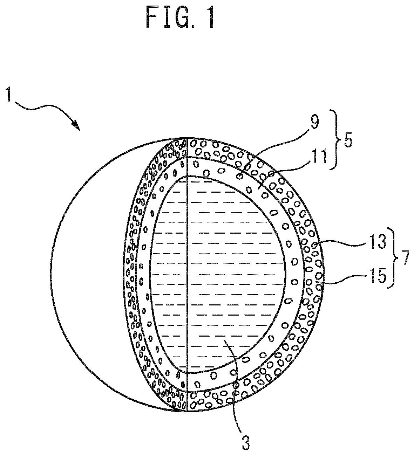

Fig. 1 is a diagram illustrating a water-containing capsule 1 according to one embodiment of the disclosure. A partial cross-section of the water-containing capsule 1 is shown in Fig. 1, to illustrate its internal structure. The water-containing capsule 1 shown in Fig. 1 includes a water-containing layer 3 that includes water, an inner layer 5 situated on the outer side of the water-containing layer 3 and an outer layer 7 situated on the outer side of the inner layer 5. The inner layer 5 is configured by including a lipophilic material 11 in which solid particles 9 are dispersed. The outer layer 7 is configured by including a hydrophilic material 15 in which lipophilic particles 13 are dispersed.

[Method of producing water-containing capsules]

The method of producing the water-containing capsules of the disclosure includes the following steps.

A step of preparing a first nozzle, a second nozzle disposed so as to surround the first nozzle on the outer side of the first nozzle, and a third nozzle disposed so as to surround the second nozzle on the outer side of the second nozzle.

A step of ejecting a material that is to form the water-containing layer, a heated material that is to form the inner layer and a heated material that is to form the outer layer, from the first nozzle, the second nozzle and the third nozzle, respectively, to form a water-containing pre-capsule.

A step of releasing the water-containing pre-capsule into a cooling solvent so as to cool the water-containing pre-capsule.

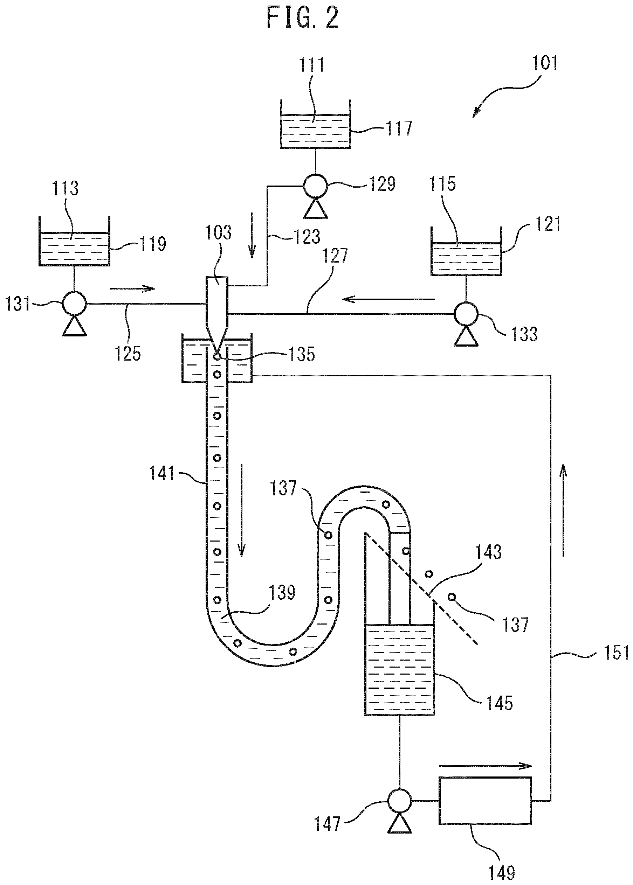

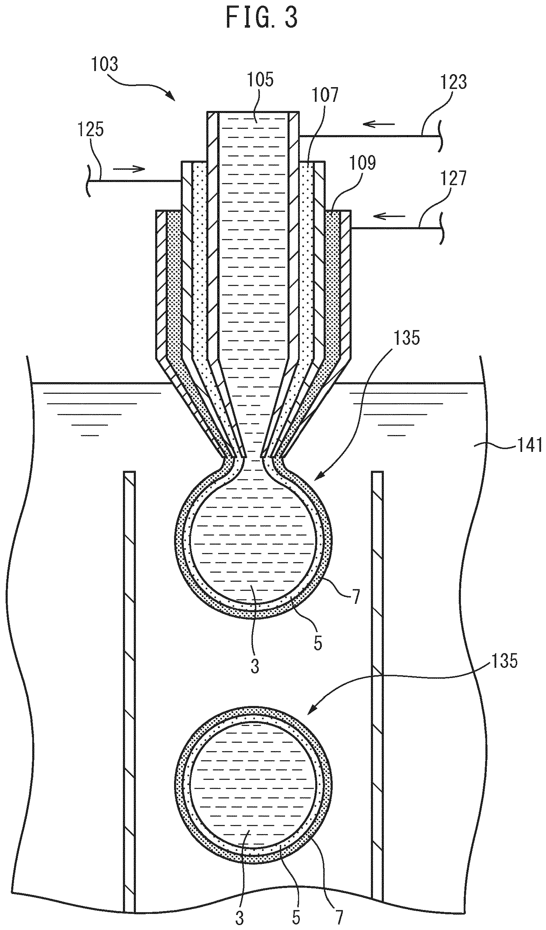

Fig. 2 and Fig. 3 are diagrams illustrating a method of producing water-containing capsules according to another embodiment of the present disclosure (hereunder also referred to as the "second embodiment"). Specifically, Fig. 2 is a schematic view of a production apparatus 101 for water-containing capsules. Fig. 3 is a magnified cross-sectional view of the vicinity of the injection nozzle 103.

The production apparatus 101 includes an injection nozzle 103, the injection nozzle 103 in turn including a first nozzle 105, a second nozzle 107 disposed so as to surround the first nozzle 105 on the outer side of the first nozzle 105, and a third nozzle 109 disposed so as to surround the second nozzle 107 on the outer side of the second nozzle 107.

The first nozzle 105 is connected with a first tank 117 that holds the material 111 that is to form the water-containing layer, a first liquid feed tube 123 that conveys the material 111 that is to form the water-containing layer, and a first metering pump 129 that adjusts the conveying speed for the material 111 that is to form the water-containing layer.

The second nozzle 107 is connected with a second tank 119 that holds a heated material 113 that is to form the inner layer, and includes a heating apparatus, a second liquid feed tube 125 that conveys the material 113 that is to form the inner layer, and a second metering pump 131 that adjusts the conveying speed for the material 113 that is to form the inner layer.

The third nozzle 109 is connected with a third tank 121 that holds a heated material 115 that is to form the outer layer and includes a heating apparatus, a third liquid feed tube 127 that conveys the material 115 that is to form the outer layer, and a third metering pump 133 that adjusts the conveying speed for the material 115 that is to form the outer layer.

The production apparatus 101 also includes a condenser tube 141 that holds a cooling solvent 139 for cooling of the water-containing pre-capsules 135 ejected from the injection nozzle 103 to form the water-containing capsules 137, a separation screen 143 that separates the water-containing capsules 137 from the cooling solvent 139, a cooling solvent-holding tank 145 that holds the cooling solvent 139, a fourth metering pump 147 that adjusts the conveying speed for the cooling solvent 139, a temperature regulator 149 that regulates the temperature of the cooling solvent 139, and a fourth liquid feed tube 151 that conveys the cooling solvent 139.

The cooling solvent 139 is circulated through the condenser tube 141, the cooling solvent-holding tank 145, the fourth metering pump 147, the temperature regulator 149 and the fourth liquid feed tube 151, the circulation speed being regulated by the fourth metering pump 147 and the temperature of the cooling solvent 139 being regulated by the temperature regulator 149.

The material 111 that is to form the water-containing layer, the heated material 113 that is to form the inner layer and the heated material 115 that is to form the outer layer, have their conveyed amounts adjusted by the first metering pump 129, the second metering pump 131 and the third metering pump 133, respectively, while being ejected from the injection nozzle 103, i.e. from the first nozzle 105, second nozzle 107 and third nozzle 109, to form the water-containing pre-capsules 135. For the second embodiment, the ejection hole of the injection nozzle 103 is situated so as to be immersed in the cooling solvent 139.

The water-containing pre-capsules 135 ejected from the injection nozzle 103 (first nozzle 105, second nozzle 107 and third nozzle 109) are cooled by the cooling solvent 139 in the condenser tube 141, forming the water-containing capsules 137. The water-containing capsules 137 are separated from the cooling solvent 139 by the separation screen 143.

The cooling solvent used in the method of producing the water-containing capsules of the disclosure is not particularly restricted so long as it is able to cool the water-containing pre-capsules, but it is preferably one that does not dissolve the hydrophilic material that is to form the outer layer.

Examples of hydrophobic solvents for the cooling solvent include hydrocarbons of 8 to 30 carbon atoms (for example, liquid paraffins, vaseline and squalene), silicone oils, lanolin, fatty acids of 4 to 30 carbon atoms, esters of fatty acids of 4 to 30 carbon atoms with sugars (such as sucrose fatty acid esters), esters of fatty acids of 4 to 30 carbon atoms and glycerol (for example, olive oil, jojoba oil, corn oil, rapeseed oil, lard, beef tallow, whale oil, castor oil, soybean oil, rice oil, rice germ oil, coconut oil, palm oil, cacao oil, avocado oil, macadamia nut oil, mink oil and turtle oil), aliphatic alcohols of 4 to 30 carbon atoms, and esters of fatty acids of 4 to 30 carbon atoms and aliphatic alcohols of 4 to 30 carbon atoms (for example, stearyl palmitate, cetyl 2-ethylhexanoate, methyl laurate, isopropyl myristate, isopropyl palmitate, 2-ethylhexyl palmitate, octyldodecyl myristate, methyl stearate, butyl stearate, 2-ethylhexyl stearate, isotridecyl stearate, myristyl myristate and stearyl stearate), as well as any desired combinations of the foregoing.

In the method of producing the water-containing capsules of the disclosure, the temperature of the heated material that is to form the inner layer is not particularly restricted, and it may be any temperature allowing conveyance of the material that is to form the inner layer, and allowing quantitative ejection of the material that is to form the inner layer from the second nozzle.

When the material that is to form the inner layer has a melting point, the temperature will usually be a temperature exceeding the melting point of the material that is to form the inner layer.

In the method of producing the water-containing capsules of the disclosure, the temperature of the heated material that is to form the outer layer is not particularly restricted, and it may be any temperature allowing conveyance of the material that is to form the outer layer, and allowing quantitative ejection of the material that is to form the outer layer from the third nozzle.

When the material that is to form the outer layer has a melting point, the temperature will usually be a temperature exceeding the melting point of the material that is to form the outer layer.

The water-containing capsules of the disclosure may be suitably used for any purpose in which water is required, and they may be used to supply moisture on-demand by applying physical irritation, such as by pressing with a finger or pinching with fingers.

Potential uses include, for example, drugs, foods, cosmetics and daily commodities (such as aromatic agents, detergents, cooling sheets, masks, eye masks and moisturizing glasses).

EXAMPLES

The present disclosure will now be explained in fuller detail by Examples, with the understanding that the disclosure is not meant to be limited to the Examples.

[Production Example 1]

The production apparatus 101 illustrated in Fig. 2 and Fig. 3 was used to produce water-containing capsules No. 1. The water-containing capsules No. 1 had a mean particle diameter of 5.0 mm.

The material for formation of the water-containing layer was a saline at a concentration of 20 mass%, prepared by dissolving sodium chloride (NaCl) in deionized water. The material for formation of the inner layer No. 1 included magnesium stearate (specific surface area: $16.2 \ m^2/g$, mean particle diameter: 3.0 $\mu$m, product of Wako Pure Chemical Industries, Ltd.) as solid particles dispersed in hydrogenated oil (extreme hydrogenated palm oil, product of Yokozeki Oil & Fat Industries Co., Ltd.) as a lipophilic material, the concentration of magnesium stearate in the material for formation of the inner layer No. 1 being 7 mass%. The material for formation of the inner layer No. 1 was heated to 80°C.

The material for formation of the outer layer No. 1 had hydrogenated oil (extreme hydrogenated palm oil, mean particle diameter: 10 $\mu$m, product of Yokozeki Oil & Fat Industries Co., Ltd.) as lipophilic particles dispersed in carrageenan (carrageenan WR-80J, product of Sansho Co., Ltd.) as the hydrophilic material. The concentration of the hydrogenated oil in the material for formation of the outer layer No. 1 was 20 mass%, and the number density of the hydrogenated oil was $540/mm^2$, as measured by the method described herein. The material for formation of the outer layer No. 1 was heated to 80°C.

The mass ratio of the water-containing layer, inner layer and outer layer in the water-containing capsules No. 1 was 50.0:40.0:10.0.

[Production Examples 2 to 5]

Water-containing capsules No. 2 to 5 were produced in the same manner as Production Example 1, except that the material for formation of the water-containing layer, the material for formation of the inner layer and the material for formation of the outer layer were changed as shown in Table 1. The compositions of each of the water-containing capsules No. 2 to 5 are shown in Table 1.

The beeswax used as the lipophilic material and lipophilic particles in Table 1 was made by Miki Chemical Industry Co., Ltd., and the nano clay used as the lipophilic particles was a standard clay sample of the Clay Science Society of Japan (mean particle diameter: 3 $\mu$m).

[Comparative Production Examples 1 to 4]

Water-containing capsules No. 6 to 9 were produced in the same manner as Production Example 1, except that the material for formation of the water-containing layer, the material for formation of the inner layer and the material for formation of the outer layer were changed as shown in Table 1. The compositions of the water-containing capsules No. 6 to 9 are shown in Table 1.

The PP of water-containing capsules No. 8 were polypropylene particles (FLOBLEN by Sumitomo Seika Chemicals Co., Ltd., mean particle diameter: 5 $\mu$m).

[Table 1]

Table 1

| Example No. | Example 1 | Example 2 | Example 3 | Example 4 | Example 5 | Comp. Example 1 | Comp. Example 2 | Comp. Example 3 | Comp. Example 4 |
|---|---|---|---|---|---|---|---|---|---|
| Water-containing capsules No. | 1 | 2 | 3 | 4 | 5 | 6 | 7 | 8 | 9 |
| Particle diameter (mm) | 5.0 | 5.0 | 5.0 | 5.0 | 5.0 | 5.0 | 5.0 | 5.0 | 5.0 |
| Water layer | | | | | | | | | |
| Composition | Water+NaCl | Water | Water | Water | Water | Water+NaCl | Water | Water | Water |
| Inner layer | | | | | | | | | |
| Lipophilic material | Hydrogenated oil | Hydrogenated oil | Beeswax | Extreme hydrogenated palm oil | Beeswax | Hydrogenated oil | Hydrogenated oil | Hydrogenated oil | Hydrogenated oil |
| Solid particles | Mg stearate | - | - | - | - | - | Mg stearate | PP | - |
| Lipophilic material/solid particles (mass ratio) | 93/7 | 100/0 | 100/0 | 100/0 | 100/0 | 100/0 | 93/7 | 99/1 | 100/0 |
| Outer layer | | | | | | | | | |
| Hydrophilic material | Carrageenan | Carrageenan | Carrageenan | Carrageenan | Carrageenan | Carrageenan | Carrageenan | Carrageenan | Carrageenan |
| Lipophilic particles or water-insoluble particles | Extreme hydrogenated palm oil | Extreme hydrogenated palm oil | Extreme hydrogenated palm oil | Beeswax | Nano clay | - | - | - | - |
| Mean particle diameter of lipophilic particles ($\mu$m) | 8.0 | 8.0 | 8.0 | 8.0 | 8.0 | - | - | - | - |

EP 3 572 062 B1

(continued)

| Example No. | Example 1 | Example 2 | Example 3 | Example 4 | Example 5 | Comp. Example 1 | Comp. Example 2 | Comp. Example 3 | Comp. Example 4 |
|---|---|---|---|---|---|---|---|---|---|
| Hydrophilic material/ particle (mass ratio) | 80/20 | 80/20 | 80/20 | 80/20 | 99/1 | 100/0 | 100/0 | 100/0 | 100/0 |
| Number density (num/mm²) | 540 | 360 | 360 | 400 | 41 | - | - | - | - |
| Water-containing capsules | | | | | | | | | |
| Water layer/ inner layer/ outer layer (mass ratio) | 50.0/40.0 /10.0 | 54.2/35.8 /10.0 | 53.7/36.6 /9.7 | 53.7/36.6 /9.7 | 53.7/36.6 /9.7 | 50.0/40.0 /10.0 | 50.0/40.0 /10.0 | 61.6/28.4 /10.0 | 50.0/40.0 /10.0 |
| Water transpiration speed (μg/day) | 16 | 28 | 12 | 15 | 16 | 319 | 35 | 56 | 54 |

[Examples 1 to 5 and Comparative Examples 1 to 4]

Water-containing capsules No. 1 to No. 9 were provided for a water transpiration test. The results are shown in Table 1.

[Water transpiration test]

(1) Each of the water-containing capsules No. 1 to No. 9 were placed in an open container in a constant temperature and humidity room at a temperature of 22±3°C and humidity of 60±5% RH, without overlapping of the particles, and were allowed to stand for 3 hours.

(2) For each of water-containing capsules No. 1 to No. 9, 20 randomly selected particles were placed on a plastic dish without overlapping, the initial total mass: $M_0$ (mg) of the plastic dish and the 20 particles was measured, and measurement of the water transpiration speed was carried out.

(3) For each of the water-containing capsules No. 1 to No. 9, the total mass $M_{nday}$ (mg) of the plastic dish and the 20 particles was monitored every n days after starting measurement of the water transpiration speed (where n is a multiple of 7), for a maximum of 12 months. For samples with severe moisture reduction, the measurement was terminated as appropriate.

(4) The data were plotted with time (days) as the abscissa and total mass of the plastic dish and 20 particles as the ordinate, and approximated to a primary expression by the least square method, and the absolute value of the slope divided by 20 was used as the water transpiration speed ($\mu$g/day). A larger value for the water transpiration speed indicates more rapid transpiration of water.

Table 1 shows that the water-containing capsules No. 1 to No. 5 had slower water transpiration speeds, i.e. more inhibited transpiration of water in the water-containing layer, compared to the water-containing capsules No. 6 to No. 9.

In addition, during any selected period of the water transpiration test, all of the water-containing capsules No. 1 to No. 5 collapsed without destruction when pinched by fingers, allowing outward release of the water in the water-containing layer.

REFERENCE SIGNS LIST

1 Water-containing capsule
3 Water-containing layer
5 Inner layer
7 Outer layer
9 Solid particle
11 Lipophilic material
13 Lipophilic particle
15 Hydrophilic material
101 Production apparatus
103 Injection nozzle
105 First nozzle
107 Second nozzle
109 Third nozzle
111 Material for formation of water-containing layer
113 Material for formation of inner layer
115 Material for formation of outer layer
117 First tank
119 Second tank
121 Third tank
123 First liquid feed tube
125 Second liquid feed tube
127 Third liquid feed tube
129 First metering pump

131 Second metering pump
133 Third metering pump
135 Water-containing pre-capsule
137 Water-containing capsule
139 Cooling solvent
141 Condenser tube
143 Separation screen
145 Cooling solvent-holding tank
147 Fourth metering pump
149 Temperature regulator
151 Fourth liquid feed tube

**Claims**

1. A water-containing capsule comprising a water-containing layer that includes water, an inner layer situated on an outer side of the water-containing layer and an outer layer situated on an outer side of the inner layer, wherein

   the inner layer is configured by including a lipophilic material,
   the outer layer is configured by including a hydrophilic material in which lipophilic particles or water-insoluble particles are dispersed, and
   the lipophilic material includes at least one of fats and oils, fatty acid, sugar fatty acid ester, aromatic hydrocarbon, chain ether, higher fatty acid ester, higher alcohol, and hydrogenated form of the foregoing, and
   components for the water-insoluble particles include at least one of metal oxide, metal carboxylate, and clay mineral.

2. The water-containing capsule according to claim 1, wherein the inner layer further includes solid particles,

   the solid particles are dispersed in the lipophilic material, and
   components for the solid particles include at least one of metal oxide, metal carbonate, metal carboxylate, clay mineral, and polymer.

3. The water-containing capsule according to claim 2, wherein the solid particles are nonporous particles.

4. The water-containing capsule according to any one of claims 1 to 3, wherein the water-containing layer further includes a component that lowers a water vapor pressure.

5. The water-containing capsule according to any one of claims 1 to 4, wherein the outer layer includes the lipophilic particles or the water-insoluble particles in a proportion of 1 to 30 mass%.

6. The water-containing capsule according to any one of claims 1 to 5, wherein the lipophilic particles or the water-insoluble particles have a mean particle diameter of 1 to 120 $\mu$m, wherein the mean particle diameter is calculated with the following steps:

   - providing a digital microscope, with a constant temperature and humidity room at temperature: $20 \pm 5°C$ and humidity: $65 \pm 5\%$ RH.
   - placing a particle sample in the constant temperature and humidity room for 3 hours;
   - photographing the sample with said digital microscope;
   - measuring a particle projected area (A) with a predetermined software included in the digital microscope and calculating a circle equivalent diameter of each particle (CD) by applying the following formula:

$$CD = 2\sqrt{\frac{A}{\pi}}$$

   - creating with said software a particle size distribution for the circle equivalent diameter (CD) of 1,000 particles, determining a median diameter ($^{50}D_p$) from the particle size distribution and recording it as the mean particle diameter of the particles.

7. The water-containing capsule according to any one of claims 1 to 6, wherein the outer layer includes the lipophilic particles or the water-insoluble particles in a number density of 100 to 2,000/mm$^2$, wherein the number density is measured in the following manner:

- cutting a perimeter of a water-containing capsule on a prescribed plane so as to include only the outer layer, to prepare a sample;
- placing the sample on slide glass and placing another cover glass over it to create a preparation;
- observing the sample with a microscope at a focal depth of 5 $\mu$m, and counting the number of lipophilic particles or water-insoluble particles within a prescribed area.

8. A method of producing the water-containing capsule according to any one of claims 1 to 7, the method comprising:

a step of preparing a first nozzle, a second nozzle disposed so as to surround the first nozzle on an outer side of the first nozzle, and a third nozzle disposed so as to surround the second nozzle on an outer side of the second nozzle,
a step of ejecting a material that is to form the water-containing layer, a heated material that is to form the inner layer and a heated material that is to form the outer layer, from the first nozzle, the second nozzle and the third nozzle, respectively, to form a water-containing pre-capsule, and
a step of releasing the water-containing pre-capsule into a cooling solvent so as to cool the water-containing pre-capsule.

**Patentansprüche**

1. Wasserhaltige Kapsel, umfassend eine wasserhaltige Schicht, die Wasser beinhaltet, eine innere Schicht, die sich auf einer Außenseite der wasserhaltigen Schicht befindet, und eine äußere Schicht, die sich auf einer Außenseite der inneren Schicht befindet, wobei

die innere Schicht konfiguriert ist, um ein lipophiles Material zu beinhalten,
die äußere Schicht konfiguriert ist, um ein hydrophiles Material zu beinhalten, in dem lipophile Partikel oder wasserunlösliche Partikel dispergiert sind, und
das lipophile Material mindestens eines von Fetten und Ölen, Fettsäure, Zuckerfettsäureester, aromatischem Kohlenwasserstoff, Kettenether, höherem Fettsäureester, höheren Alkoholen und hydrierten Formen der vorgenannten Stoffe beinhaltet, und
Bestandteile für die wasserunlöslichen Partikel mindestens eines von Metalloxid, ein Metallcarboxylat oder Tonmineral beinhalten.

2. Wasserhaltige Kapsel nach Anspruch 1, wobei die innere Schicht feste Partikel beinhaltet,

die festen Partikel in dem lipophilen Material dispergiert sind, und
Bestandteile für die wasserunlöslichen Partikel mindestens eines von Metalloxid, Metallcarbonat, Metallcarboxylat, Tonmineral und Polymer beinhalten.

3. Wasserhaltige Kapsel nach Anspruch 2, wobei die festen Partikel nicht-poröse Partikel sind.

4. Wasserhaltige Kapsel nach einem der Ansprüche 1 bis 3, wobei die wasserhaltige Schicht außerdem einen Bestandteil beinhaltet, der den Wasserdampfdruck senkt.

5. Wasserhaltige Kapsel nach einem der Ansprüche 1 bis 4, wobei die äußere Schicht die lipophilen Partikel oder die wasserunlöslichen Partikel in einem Anteil von 1 bis 30 Massenprozent beinhaltet.

6. Wasserhaltige Kapsel nach einem der Ansprüche 1 bis 5, wobei die lipophilen Partikel oder die wasserunlöslichen Partikel einen mittleren Partikeldurchmesser von 1 bis 120 $\mu$m aufweisen, wobei der mittlere Partikeldurchmesser mit den folgenden Schritten berechnet wird:

- Bereitstellen eines digitalen Mikroskops, mit einem Raum konstanter Temperatur und Luftfeuchtigkeit bei Temperatur: 20 $\pm$5 °C und Luftfeuchtigkeit: 65 +5 % RH.
- Platzieren einer Partikelprobe in dem Raum mit konstanter Temperatur und Luftfeuchtigkeit über 3 Stunden;

- Fotografieren der Probe mit dem Digitalmikroskop;
- Messen einer projizierten Partikelfläche (A) mit einer vorbestimmten Software, die in dem digitalen Mikroskop beinhaltet ist, und Berechnen eines kreisäquivalenten Durchmessers von jedem Partikel (CD) durch Anwenden der folgenden Formel:

$$CD = 2\sqrt{\frac{A}{\pi}}$$

- Erstellen einer Partikelgrößenverteilung für den kreisäquivalenten Durchmesser (CD) von 1000 Partikeln mit der Software, Bestimmen eines Median-Durchmessers ($^{50}D_p$) aus der Partikelgrößenverteilung und Aufzeichnen desselben als mittleren Partikeldurchmesser der Partikel.

7. Wasserhaltige Kapsel nach einem der Ansprüche 1 bis 6, wobei die äußere Schicht die lipophilen Partikel oder die wasserunlöslichen Partikel in einer Zahlendichte von 100 bis 2000/mm$^2$ beinhaltet, wobei die Zahlendichte auf folgende Weise gemessen wird:

- Schneiden eines Umfangs einer wasserhaltigen Kapsel auf einer vorgegebenen Ebene, sodass nur die äußere Schicht eingeschlossen ist, um eine Probe herzustellen;
- Platzieren der Probe auf ein Objektträgerglas und Auflegen eines weiteren Deckglases, um ein Präparat zu erstellen;
- Beobachten der Probe mit einem Mikroskop bei einer Fokustiefe von 5 $\mu$m und Zählen der Anzahl der lipophilen oder wasserunlöslichen Partikel innerhalb eines vorgegebenen Bereichs.

8. Verfahren zum Herstellen der wasserhaltigen Kapsel nach einem der Ansprüche 1 bis 7, das Verfahren umfassend:

einen Schritt eines Herstellens einer ersten Düse, einer zweiten Düse, die angeordnet ist, um die erste Düse an einer Außenseite der ersten Düse zu umgeben, und einer dritten Düse, die angeordnet ist, um die zweite Düse an einer Außenseite der zweiten Düse zu umgeben,
einen Schritt eines Ausstoßens eines Materials, das die wasserhaltige Schicht bilden soll, eines erhitzten Materials, das die innere Schicht bilden soll, und eines erhitzten Materials, das die äußere Schicht bilden soll, aus der ersten Düse, der zweiten Düse bzw. der dritten Düse, um eine wasserhaltige Vorkapsel zu bilden, und
einen Schritt eines Freisetzens der wasserhaltigen Vorkapsel in ein Kühllösungsmittel, um die wasserhaltige Vorkapsel zu kühlen.

## Revendications

1. Capsule contenant de l'eau comprenant une couche contenant de l'eau qui comprend de l'eau, une couche interne située sur un côté extérieur de la couche contenant de l'eau et une couche externe située sur un côté extérieur de la couche interne, dans laquelle

la couche interne est configurée en incluant un matériau lipophile,
la couche externe est configurée en incluant un matériau hydrophile dans lequel sont dispersées des particules lipophiles ou des particules insolubles dans l'eau, et
le matériau lipophile comprend au moins l'un des éléments suivants : graisses et huiles, acide gras, ester d'acide gras de sucre, hydrocarbure aromatique, éther sous forme de chaîne, ester d'acide gras supérieur, alcool supérieur et forme hydrogénée de ce qui précède, et
les composants des particules insolubles dans l'eau comprennent au moins un oxyde métallique, un carboxylate métallique et un minéral argileux.

2. Capsule contenant de l'eau selon la revendication 1, dans laquelle la couche interne comprend en outre des particules solides,

les particules solides sont dispersées dans le matériau lipophile, et
les composants des particules solides comprennent au moins un oxyde métallique, un carbonate métallique, un carboxylate métallique, un minéral argileux et un polymère.

**3.** Capsule contenant de l'eau selon la revendication 2, dans laquelle les particules solides sont des particules non poreuses.

**4.** Capsule contenant de l'eau selon l'une quelconque des revendications 1 à 3, dans laquelle la couche contenant de l'eau comprend en outre un composant qui abaisse une pression de vapeur d'eau.

**5.** Capsule contenant de l'eau selon l'une quelconque des revendications 1 à 4, dans laquelle la couche externe comprend les particules lipophiles ou les particules insolubles dans l'eau dans une proportion de 1 à 30 % en masse.

**6.** Capsule contenant de l'eau selon l'une quelconque des revendications 1 à 5, dans laquelle les particules lipophiles ou les particules insolubles dans l'eau ont un diamètre moyen de particule de 1 à 120 μm, dans laquelle le diamètre moyen de particule est calculé selon les étapes suivantes :

- fournir un microscope numérique, avec une pièce à température et humidité constantes à la température : 20±5 °C et l'humidité : 65+5 % HR.
- placer un échantillon de particules dans la pièce à température et humidité constantes pendant 3 heures ;
- photographier l'échantillon avec ledit microscope numérique ;
- mesurer une surface projetée de particules (A) avec un logiciel prédéterminé inclus dans le microscope numérique et calculer un diamètre équivalent circulaire de chaque particule (CD) en appliquant la formule suivante :

$$\text{CD} = 2\sqrt{\frac{A}{\pi}}$$

- créer avec ledit logiciel une distribution de taille de particule pour le diamètre équivalent circulaire (CD) de 1 000 particules, déterminer un diamètre médian ($^{50}D_p$) à partir de la distribution de taille de particule et l'enregistrer comme le diamètre moyen de particule des particules,

**7.** Capsule contenant de l'eau selon l'une quelconque des revendications 1 à 6, dans laquelle la couche externe comprend les particules lipophiles ou les particules insolubles dans l'eau dans une densité de particules de 100 à 2 000/mm$^2$, dans laquelle la densité de particules est mesurée de la manière suivante :

- découpe d'un périmètre d'une capsule contenant de l'eau sur un plan prescrit de manière à n'inclure que la couche extérieure, pour préparer un échantillon ;
- placement de l'échantillon sur le verre de la lame et placement d'un autre verre de couverture par-dessus pour créer une préparation ;
- observation de l'échantillon avec un microscope à une profondeur de foyer de 5 μm, et comptage du nombre de particules lipophiles ou de particules insolubles dans l'eau dans une zone prescrite.

**8.** Procédé de production de la capsule contenant de l'eau selon l'une quelconque des revendications 1 à 7, le procédé comprenant :

une étape de préparation d'une première buse, d'une deuxième buse disposée de façon à entourer la première buse sur un côté extérieur de la première buse, et d'une troisième buse disposée de façon à entourer la deuxième buse sur un côté extérieur de la deuxième buse,
une étape d'éjection d'un matériau qui doit former la couche contenant de l'eau, d'un matériau chauffé qui doit former la couche interne et d'un matériau chauffé qui doit former la couche externe, de la première buse, de la deuxième buse et de la troisième buse, respectivement, pour former une pré-capsule contenant de l'eau, et

une étape consistant à libérer la pré-capsule contenant de l'eau dans un solvant de refroidissement de façon à refroidir la pré-capsule contenant de l'eau.

FIG. 1

# FIG. 2

# FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015115072 A
- EP 2749272 A1